# EUROPÄISCHE PATENTSCHRIFT

(11) **EP 0 780 390 B1**
(45) Veröffentlichungstag und Bekanntmachung des Hinweises auf die Patenterteilung: **31.07.2002**
(21) Anmeldenummer: 96119134.3
(22) Anmeldetag: 29.11.1996
(51) Int. Cl.: C07D 471/04, A61K 31/47

(54) **Kristallmodifikation des CDCH, Verfahren zu dessen Herstellung und diese enthaltende pharmazeutische Zubereitungen**
Crystalline modification of CDCH, process for its preparation and pharmaceutical compositions containing it
Modification cristalline du CDCH, procédé pour sa préparation et compositions pharmaceutiques la contenant

(30) Priorität: 12.12.1995 DE 19546249
(43) Veröffentlichungstag der Anmeldung: 25.06.1997
(73) Patentinhaber: BAYER AG, 51368 Leverkusen (DE)
(72) Erfinder: Grunenberg, Alfons, Dr., 41539 Dormagen (DE); Bosché, Patrick, Dr., 51519 Odenthal (DE)

(56) Entgegenhaltungen:
- EP-A- 0 230 274
- EP-A- 0 241 206
- EP-A- 0 342 649
- EP-A- 0 550 903
- EP-A- 0 591 808
- EP-A- 0 603 887
- EP-A- 0 629 621
- EP-A- 0 643 058

## Beschreibung

Die Erfindung betrifft das neue Monohydrat des 1-Cyclopropyl-7-([S,S]-2,8-diazabicyclo[4.3.0]non-8-yl)6-fluor-1,4-dihydro-8-methoxy-4-oxo-3-chinolincarbonsäure Hydrochlorid (CDCH), ein Verfahren zu dessen Herstelllung und pharmazeutische Zubereitungen, die dieses Monohydrat als Wirkstoff enthalten.

CDCH ist ein Chemotherapeutikum für Menschen und Tiere mit einem breiten Spektrum antibakterieller Wirkung. Der Wirkstoff kann auch im Materialschutz eingesetzt werden. CDCH zeigt geringe Toxizität und ist besonders effektiv gegen Enterobacteriacea und ganz besonders gegen Antibiotika resistente Stämme: S. aureus, Ps. aeruginosa, Enterococcus faecalis und E. coli. CDCH und dessen Herstellung als Betain ist in EP-A-550 903 und EP-A- 591 808 beschrieben.

Die EP-A 643 058 beschreibt eine Methode zur selektiven Herstellung verschiedener definierter Hydrate einer bestimmten Chinoloncarbonsäure; damit sollen die bei unkontrollierter Kristallisation anfallenden Gemische unterschiedlicher Hydrate vermieden werden.

Die EP-A 629 621 betrifft eine andere Chinoloncarbonsäure "Q-35" und ihre Überführung in ein Dihydrat, das in einer stabileren Kristallform kristallisiert.

Aus den EP-A 241 206, 230 274 und 342 649 sind Chinoloncarbonsäure-Hydrochlorid-Hydrate, nicht aber Moxifloxacin-Hydrochlorid-Hydrate bekannt, ohne dass den entsprechenden Kristallformen irgendeine vorteilhafte Eigenschaft zugeschrieben wurde.

Eine wasserfreie Form von CDCH ist die einzige Kristallmodifikation, die bisher bekannt ist. Diese Kristallmodifikation kann jedoch bei der Herstellung verschiedener Arzneiformen nicht restlos befriedigen. CDCH ist hygroskopisch und nimmt unter ungünstigen Lagerungsbedingungen und bei der galenischen Verarbeitung des Wirkstoffs zu Arzneiformen Wasser auf. Dies beeinträchtigt die Dosiergenauigkeit und Qualität der Präparate. Ursache für die physikalische Instabilität von CDCH sind nachträgliche Veränderungen in der Kristallstruktur der wasserfreien Form, wenn CDCH in wäßrigen Suspensionen oder bei Umgebungsfeuchte gelagert werden. Es ist deshalb von großer Bedeutung, eine möglichst stabile Kristallform für die Herstellung von CDCH enthaltende Arzneiformen zu verwenden.

Es wurde nun gefunden, daß CDCH in eine neue wasserhaltige, kristalline Modifikation überführt werden kann, die sich gegenüber der bekannten wasserfreien Form durch erhöhte Stabilität, insbesondere bei der Lagerung bei hohen Feuchten, auszeichnet und für die Herstellung von beständigen pharmazeutischen Präparaten sehr gut geeignet ist.

Bei der Herstellung des Monohydrats aus wäßrigen Medien kristallisiert der Wirkstoff in Form von Nadeln, die stark verfilzen. Überraschenderweise kann unter bestimmten Kristallisationsbedingungen der Kristallhabitus gezielt verändert werden. Die so entstehenden Prismen stellen eine bevorzugte Ausführungsform der vorliegenden Erfindung dar denn sie verfilzen nicht und sind bedeutend fließfähiger als das Monohydrat in Form von Nadeln. Dies hat erhebliche Vorteile bei der Herstellung von Arzneiformen. Durch die Verwendung eines nicht hygroskopischen, rieselfähigen Wirkstoffs wird eine befriedigende Dosiergenauigkeit bei der Herstellung von Arzneimitteln erreicht, die Sicherheit erhöht und somit das Risiko für den Patienten minimiert.

Gegenstand der Erfindung ist demnach das neue Monohydrat des CDCH der Formel I 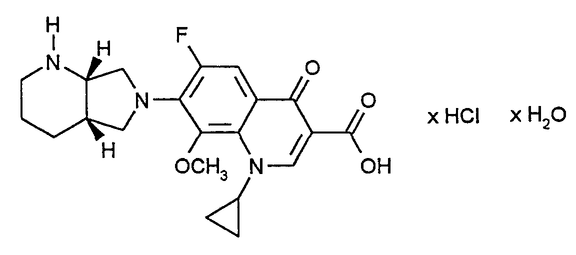 sowie ein Verfahren zu seiner Herstellung, welches dadurch gekennzeichnet ist, daß man wasserfreies, kristallines CDCH mit einer zur guten Durchmischung und Bildung des Monohydrats ausreichenden, Wassermenge bei Temperaturen unterhalb von 80°C bis zur Aufnahme des stöchiometrischen Kristallwassergehaltes und vollständigen Kristallumwandlung behandelt, die so erhaltenen Kristalle abtrennt und zur Entfernung von vorhandenem adsorbierten Wassers bis zur Gewichtskonstanz des Monohydrats trocknet. Zur Vermeidung der Bildung der wasserfreien Form sollte die Feuchte bei der Trocknung nicht geringer als 30 % r.F. sein. Das Monohydrat kristallisiert aus wasserhaltigen Medien mit einem Wasseranteil von mehr als 10 % in Form von Nadeln.

Die bevorzugte, prismatisch kristallisierende Form des Monohydrats kann dadurch erhalten werden, daß man wasserfreies, kristallines CDCH in Ethanol/Wasser-Gemischen, ganz besonders bevorzugt in Ethanol/Wasser mit max. 10 % Wasser suspendiert, wobei man bis zur Aufnahme des erforderlichen Kristallwassergehalts und vollständigen Kristallumwandlung für eine gute Durchmischung der Feststoffanteile mit der zugesetzten Wassermenge sorgt, beispielweise durch Rühren der Suspension oder Schütteln, Schwenken, Rotieren des Reaktionsgefäßes und dergleichen. Beträgt der Wassergehalt im Ethanol/Wasser-Gemisch max. 10 %, kristallisiert das Monohydrat in Form von Prismen.

Unter der Bedingung, daß die Wassermenge zur Bildung eines stöchiometrischen Monohydrats und zu einer guten Durchmischung der eingesetzten Menge an CDCH mit Wasser ausreicht, kann für die Bildung des Monohydrats in Form von Nadeln beliebig viel Wasser verwendet werden, da die Aufnahme von Kristallwasser mit der unter Kristallumwandlung verlaufenden Bildung des Monohydrats beendet ist und darüber hinaus keine weiteren Hydrate erhalten werden. Zweckmäßigerweise wird die Wassermenge so begrenzt, daß zwar eine gute Durchmischung stattfinden kann, aber keine oder geringe Löslichkeitsverluste auftreten. Die Herstellung des Monohydrats wird in bevorzugter Weise bei Raumtemperatur vorgenommen, kann jedoch auch bei erhöhter Temperatur, beispielsweise 30°C bis 60°C oder niedriger Temperatur, beispielsweise 5°C bis 20°C, durchgeführt werden. Die Herstellung des Monohydrats gelingt aus der wasserfreien Form auch bei Feuchten größer 30 % r.F. Dieses Verfahren eignet sich jedoch nicht zur Herstellung des bevorzugten, prismatisch kristallisierenden Monohydrats.

Das Abtrennen der Kristalle des Monohydrats von überschüssigem Lösemittel geschieht nach üblichen Methoden, beispielsweise durch Filtrieren, Abdekantieren, Zentrifugieren und dergleichen. Vorteihafterweise trocknet man die abgetrennten Kristalle des Monohydrats bei Raumtemperatur oder bei erhöhter Temperatur bis 50°C bei Feuchten von mindestens 30 % r.F.

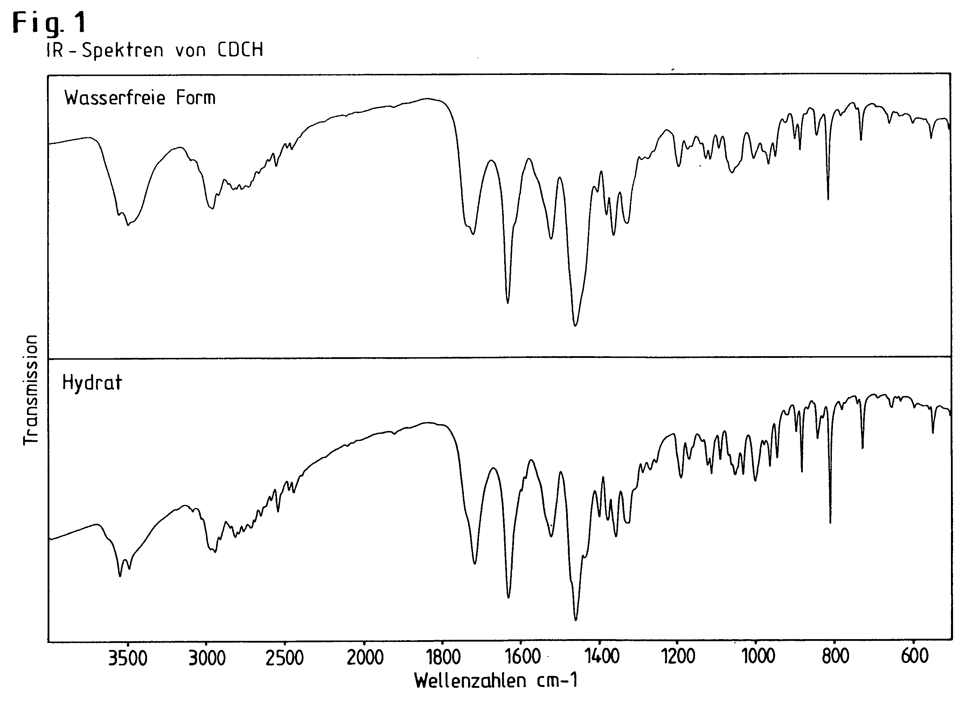

Das erfindungsgemäße Monohydrat des CDCH besitzt ein charakeristisches IR-Spektrum (Abb. 1), welches charakteristische Absorptionsbanden des Kristallwassers im Bereich der OH-Valenzschwingungen (3600 - 3100 cm⁻¹) zeigt, die bei der wasserfreien Kristallmodifikation fehlen. Es unterscheidet sich auch in anderen Frequenzbereichen vom wasserfreien CDCH, so daß auf eine völlig unterschiedliche Anordnung der Moleküle in den Kristallgittern der beiden Modifikationen geschlossen werden kann.

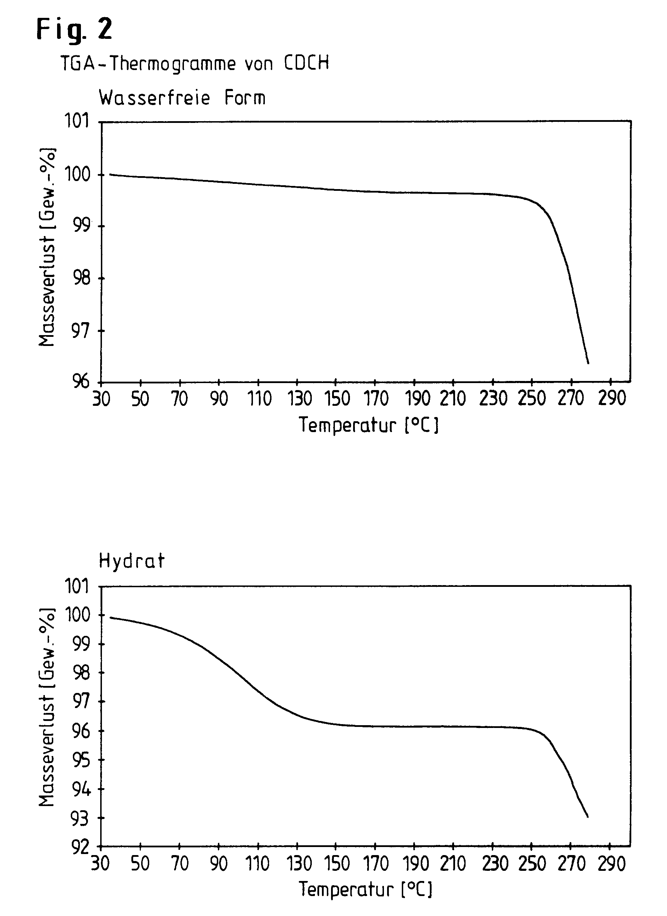

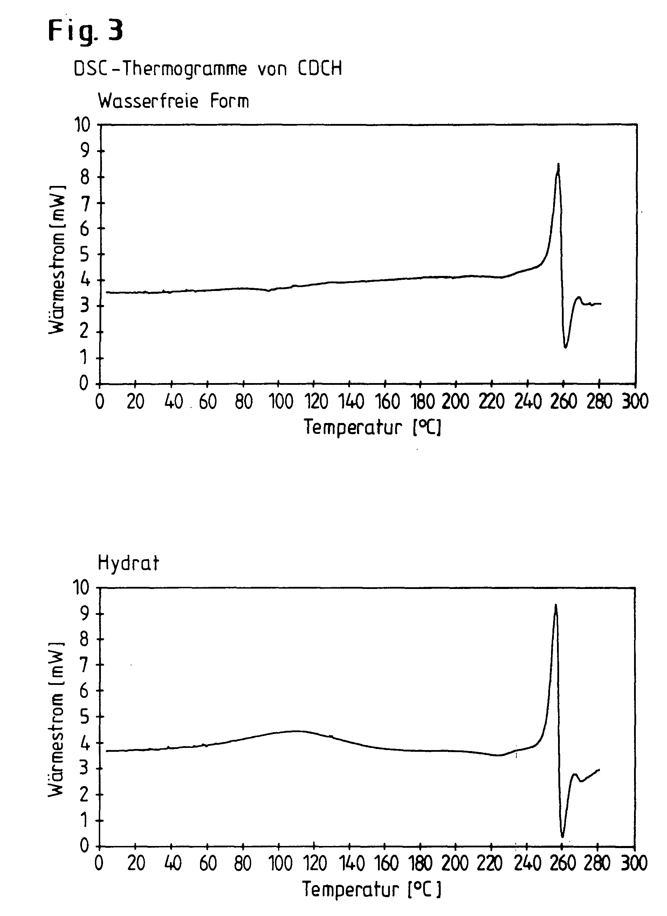

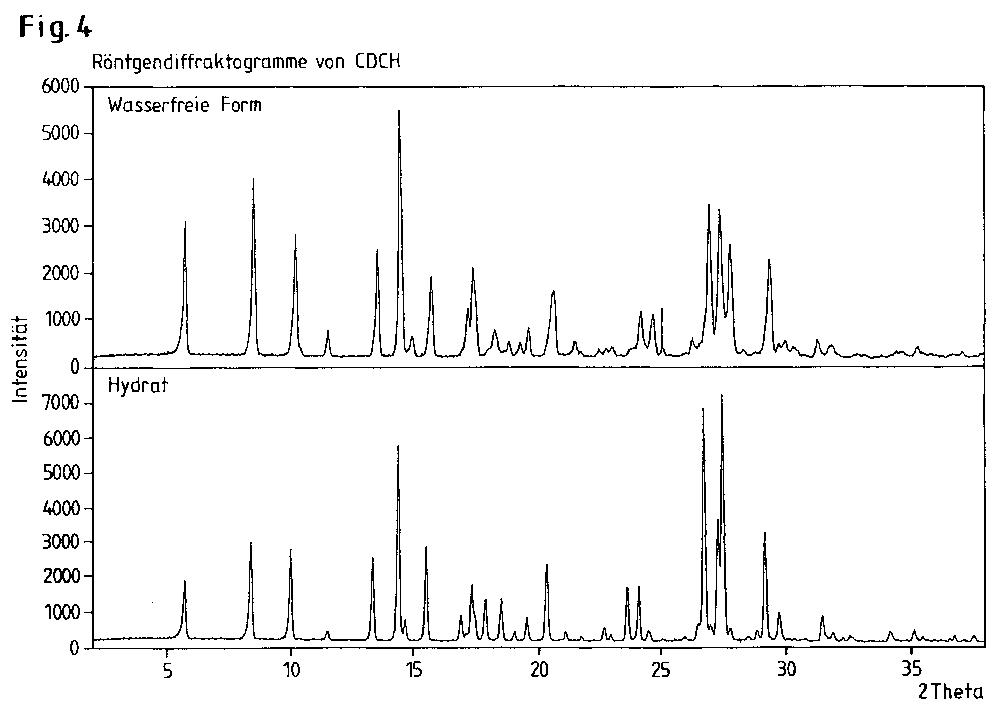

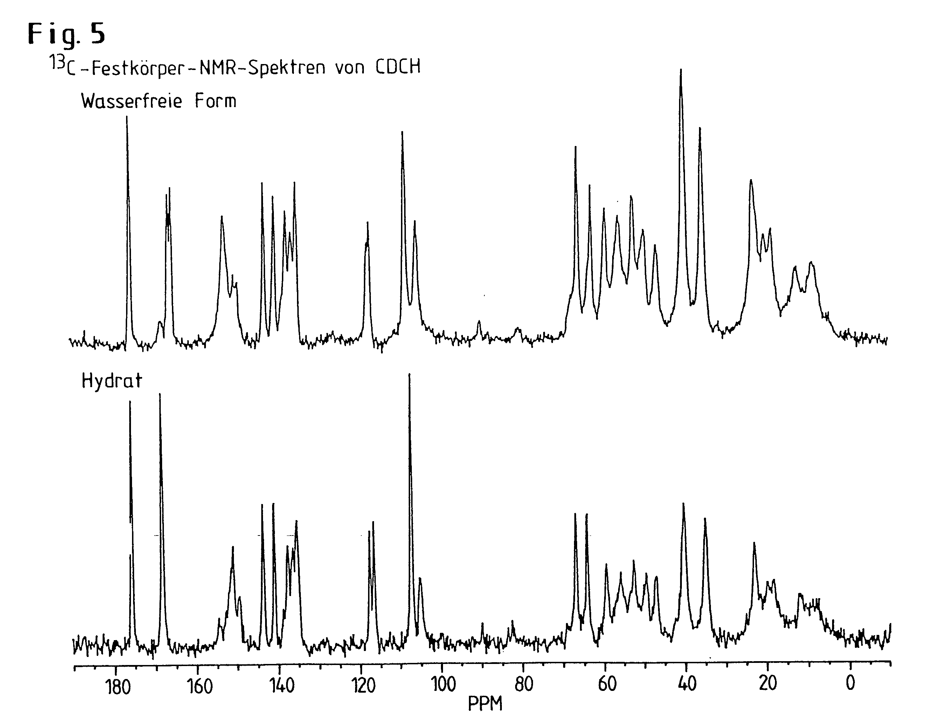

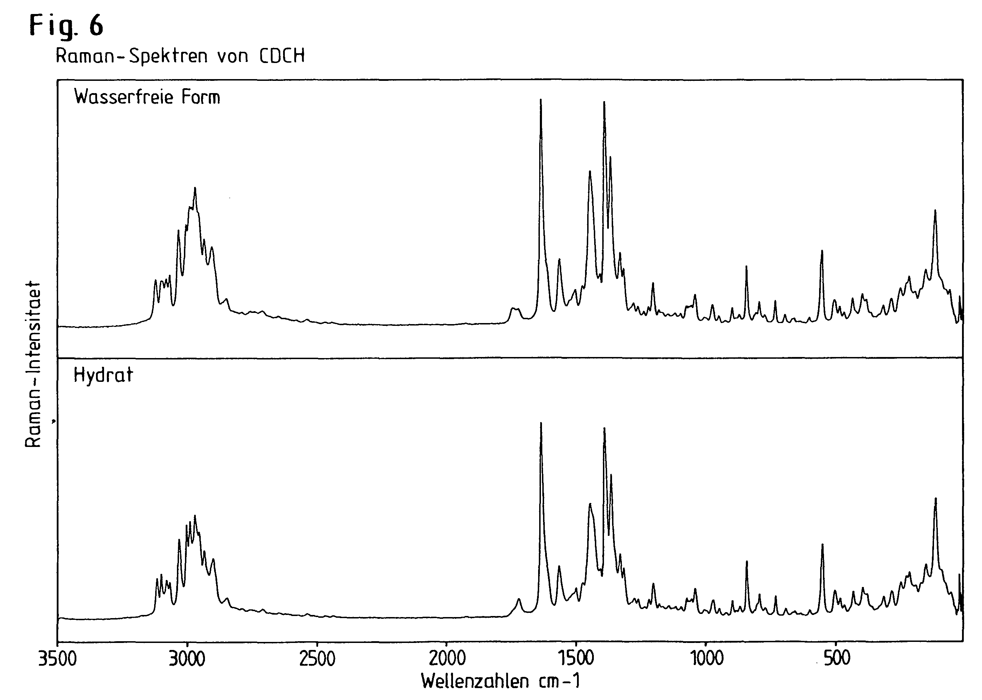

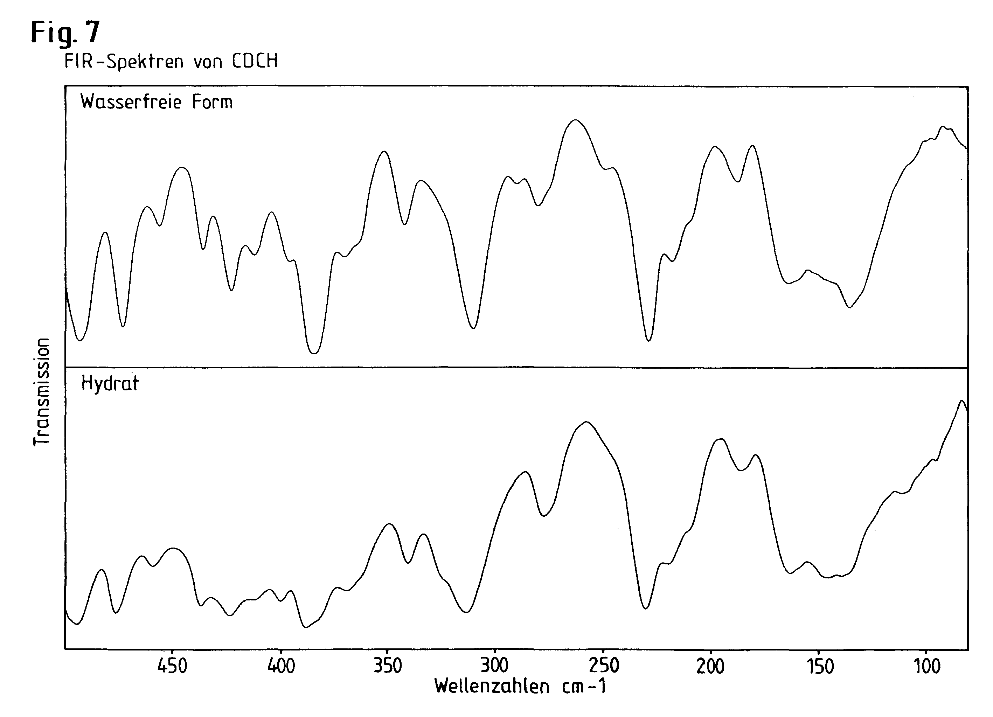

Die Bestimmung des Wassergehalts bestätigt das Vorliegen eines stöchiometrischen Monohydrats des CDCH. Der in mehreren Proben des Monohydrats bestimmte thermogravimetrische Masseverlust beträgt 1 mol Wasser (3,9 %, Abb. 2). Das mittels DSC (Differential Scanning Calorimetry) unter Atmosphärendruck aufgenomme Thermogramm des Monohydrats (Abb. 3) zeigt in Übereinstimmung mit den thermogravimetrischen Messungen die Wasserabgabe durch einen breiten endothermen Peak, der auf den Umbau des Kristallgitters des untersuchten Monohydrats, die Dissoziation von CDCH und Wasser und die Verdampfungsenthalpie des freigesetzten Kristallwassers hinweist. Die Röntgendiffraktogramme, ¹³C-NMR-, Raman- und FIR-Spektren der wasserfreien Form und des Monohydrats zeigen charakteristische Unterschiede (Abb. 4-7, Tab. 2-5): So weist z.B. das ¹³C-NMR-Spektrum einen charakteristischen Peak bei 168,1 ppm und das Röntgendiffraktogramm eine Linie bei 2Θ = 26,7 auf.

Die DSC- und TGA-Thermogramme wurden unter Verwendung von Thermoanalysengeräten (DSC 7 und TGA 7) der Fa. Perkin-Elmer erhalten. Die Röntgendiffraktogramme wurden mit einem Stoe Transmissiondiffraktometer registriert. Die IR-, FIR- und Raman-Spektren wurden mit Fourier-IR Spektrometren IFS 66 (IR), IFS 66v (FIR) und IFS 88 (Raman) der Fa. Bruker aufgenommen. Die ¹³C-Festkörper-NMR-Spektren wurden mit einem Bruker MSL 300 registriert. Die mikroskopischen Aufnahmen wurden mit einem Mikroskop Laborlux S der Fa. Leitz aufgenommen.

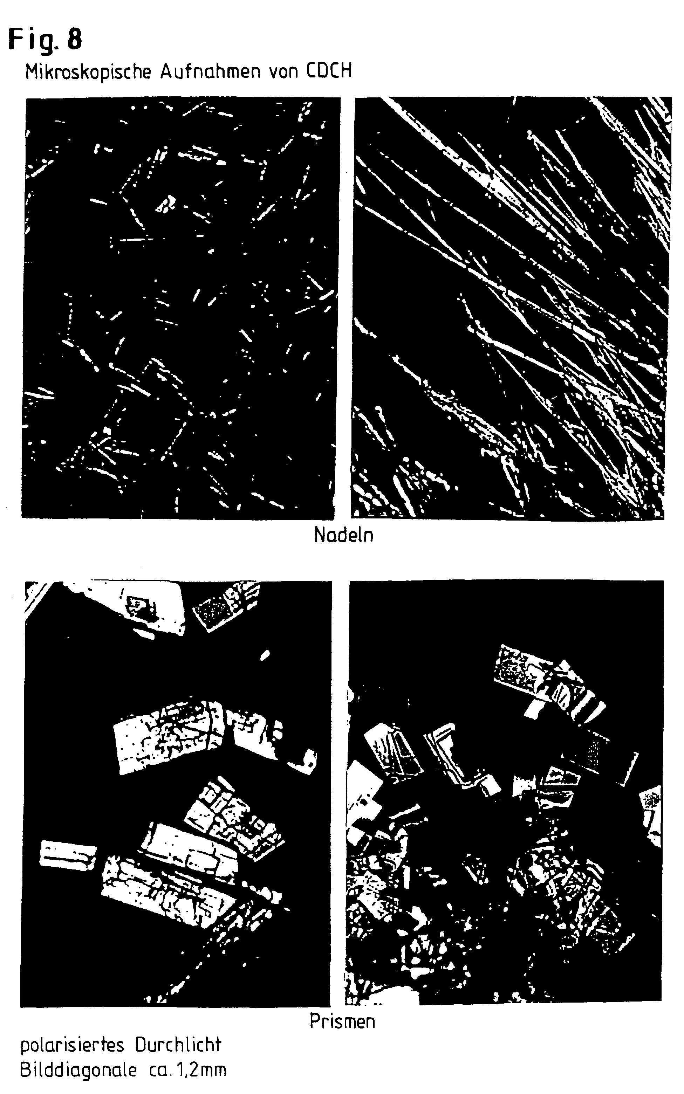

Das erfindungsgemäße Monohydrat des CDCH zeigt bei Lagerung im Vergleich zur wasserfreien Kristallmodifikation eine höhere physikalische Stabilität und ist daher für die Herstellung verschiedener Arzneiformen besser geeignet. Das bevorzugte, in Form von Prismen kristallisierende Monohydrat verleiht CDCH außerdem eine ausgezeichnete Riesel- und Fließfähigkeit, was bei der Herstellung von pharmazeutischen Zubereitungen von großem Vorteil ist (Abb. 8). Gegenstand der Erfindung sind daher auch flüssige und feste pharmazeutische Zubereitungen, die das erfindungsgemäße Monohydrat des CDCH enthalten, wie z.B. Suspensionen, Emulsionen, Tabletten, Dragees, Drageekerne, Suppositorien, Hart- oder Weichgelantinekapseln und dergleichen. Bevorzugt enthalten wäßrige Suspensionen und Tabletten für die orale Applikation das erfindungsgemäße Monohydrat, besonders bevorzugt in der prismatischen Kristallform.

CDCH kann in diesen pharmazeutischen Zubereitungen als einziger Wirkstoff vorliegen oder mit anderen antibakteriell wirksamen Substanzen kombiniert werden.

Pharmazeutische Zubereitungen können aus dem erfindungsgemäßen Monohydrat des CDCH alleine oder mit mehreren anderen Wirkstoffen kombiniert bestehen oder zusammen mit in der Galenik üblicherweise eingesetzten Hilfs- und Zusatzmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Geschmackstoffen und dergleichen zu Darreichungsformen für die orale, parenterale oder rektale Applikation formuliert sein.

Die Herstellung der pharmazeutischen Zubereitungen erfolgt in an sich bekannter Weise, beispielsweise durch Mischen, Einrühren, Suspendieren, Dispergieren, Emulgieren und dergleichen der Wirkstoffe mit bzw. in den pharmazeutischen Hilfsstoffen und Verarbeitung zu pharmazeutisch geeigneten Darreichungsformen für die orale, parenterale oder rektale Applikation.

### Herstellung von kristallinem CDCH (Nadeln, Prismen)

### Beispiel 1 (Prismen)

1 g wasserfreies CDCH wird in 150 ml absolutem Ethanol gelöst und die Lösung filtriert. Die Lösung wird bei 60 °C bis zum vollständigen Verdunsten des Lösungsmittels getempert. Die ausgefallenen Kristalle werden bei Raumtemperatur/-Umgebungsfeuchte getrocknet.

### Beispiel 2 (Prismen)

0,1 g wasserfreies CDCH werden in 10 ml Ethanol (10 % Wasseranteil) gelöst. Die Lösung wird bei 60°C bis zum vollständigen Verdunsten des Lösungsmittels getempert. Die ausgefallenen Kristalle werden bei Raumtemperatur/Umgebungsfeuchte getrocknet.

### Beispiel 3 (Prismen)

4 g wasserfreies CDCH werden in 300 ml Ethanol (96 %) gelöst. Das Lösungsmittel wird im Rotationsverdampfer bei 60°C und 120 mbar abdestilliert. Die Kristalle werden im Vakuumtrockenschrank 2 h bei 80 mbar und 105°C getrocknet und anschließend Umgebungsfeuchte ausgesetzt.

### Beispiel 4 (Nadeln)

0,3 g wasserfreies CDCH werden in 6 ml Wasser:Ethanol (1:1) gelöst. Die Lösung wird bei 70°C bis zum vollständigen Verdunsten des Lösungsmittels getempert. Die ausgefallenen Kristalle werden bei Raumtemperatur im Vakuum getrocknet und anschließend bei RT/85 % r. F. über Nacht stehengelassen.

### Beispiel 5 (Nadeln)

0,1 g wasserfreies CDCH werden in 5 ml Methanol gelöst. Die Lösung wird bei RT bis zum vollständigen Verdunsten des Lösungsmittels stehengelassen. Die Kristalle werden bei Raumtemperatur im Vakuum getrocknet und anschließend bei RT/85 % r. F. über Nacht stehengelassen.

### Beispiel 6 (Nadeln)

0,1 g wasserfreies CDCH werden in 5 ml Wasser gelöst. Die Lösung wird bei RT bis zum vollständigen Verdunsten des Lösungsmittels stehengelassen. Die Kristalle werden bei Raumtemperatur im Vakuum getrocknet und anschließend bei RT/85 % r. F. über Nacht stehengelassen.

### Beispiel 7

25,1 g CDCH-Monohydrat (Prismen), 3,3 g Avicel PH 101 und 1,7 g Maisstärke werden im Mischgranulator gemischt und anschließend mit 13 g Wasser granuliert. Nach Raspeln (4 mm) wird das Granulat im Mini-Wirbelschichttrockner getrocknet (Zulufttemperatur 80°C) und über ein 0,8 mm Sieb gesiebt. Die Nachmischung erfolgt mit 0,19 g Ac-Di-Sol und 0,01 g Magnesiumstearat. Anschließend erfolgt die Verpressung auf einer Exzenterpresse (Tablettenformat 5,5 r 9, Tablettengewicht 68,5 mg).

### Beispiel 8

196,6 g mikronisiertes CDCH-Monohydrat (Nadeln) werden mit 88 g Avicel im Mischgranulator gemischt (Pulvermischung). 3,6 g PVP 25 werden in 97,2 g Wasser gelöst (Granulationsflüssigkeit). Die Pulvermischung wird mit der Granulationsflüssigkeit granuliert. Nach Raspeln (3 mm) wird das Granulat im Schnelltrockner (Zulufttemperatur 90°C) getrocknet und über ein 1 mm Sieb gesiebt. Die Nachmischung erfolgt mit 1,8 g Ac-Di-Sol und 0,1 g Magnesiumstearat. Anschließend erfolgt die Verpressung auf einer Rundläuferpresse (Tablettenformat 5,5 r 9, Tablettengewicht 83,4 mg).

**Tab. 1:**

| IR-Spektroskopie | |
|---|---|
| wasserfreie Form [cm-1] | Hydrat [cm-1] |
| 722 | 722 |
| 804 | 804 |
| 834 | 835 |
| 938 | 875 |
| 957 | 938 |
| 994 | 994 |
| 1048 | 1045 |
| 1186 | 1082 |
| 1319 | 1163 |
| 1354 | 1184 |
| 1372 | 1319 |
| 1453 | 1352 |
| 1513 | 1372 |
| 1622 | 1394 |
| 1709 | 1432 |
| 2427 | 1456 |
| 2524 | 1517 |
| 2700 | 1624 |
| 2929 | 1709 |
| 3469 | 2427 |
| 3527 | 2456 |
| | 2524 |
| | 2634 |
| | 2925 |
| | 2698 |
| | 2745 |
| | 2893 |
| | 2925 |
| | 3472 |
| | 3530 |

**Tab. 2:**

| Röntgendiffraktometrie | |
|---|---|
| wasserfreie Form [2 Theta] | Hydrat [2 Theta] |
| 5,8 | 5,8 |
| 8,6 | 8,5 |
| 10,3 | 10,1 |
| 11,6 | 11,6 |
| 13,6 | 13,4 |
| 14,5 | 14,5 |
| 15,0 | 14,8 |
| 15,8 | 15,6 |
| 17,3 | 17,0 |
| 17,5 | 17,2 |
| 18,3 | 17,4 |
| 18,9 | 17,5 |
| 19,3 | 17,9 |
| 19,6 | 18,6 |
| 20,6 | 19,1 |
| 21,5 | 19,6 |
| 22,5 | 20,4 |
| 22,8 | 21,1 |
| 23,0 | 21,8 |
| 23,8 | 22,7 |
| 24,2 | 23,0 |
| 24,7 | 23,6 |
| 25,0 | 24,1 |
| 26,3 | 24,5 |
| 27,0 | 26,5 |
| 27,4 | 26,7 |
| 27,8 | 27,0 |
| 28,2 | 27,3 |
| 29,4 | 27,5 |
| 29,7 | 27,8 |
| 30,0 | 28,5 |
| 30,3 | 28,9 |
| 31,3 | 29,2 |
| 31,8 | 29,7 |
| 34,5 | 31,4 |
| 35,3 | 31,9 |
| 37,1 | 32,3 |
| | 32,6 |
| | 34,2 |
| | 35,1 |
| | 35,5 |
| | 36,8 |
| | 37,5 |

**Tab. 3:**

| ¹³C-Festkörper-NMR-Spektroskopie | |
|---|---|
| wasserfreie Form [ppm] | Hydrat [ppm] |
| 8,5 | 7,7 |
| 12,3 | 8,3 |
| 14,1 | 9,0 |
| 18,2 | 10,8 |
| 20,0 | 12,1 |
| 22,8 | 18,2 |
| 35,2 | 19,8 |
| 39,7 | 22,9 |
| 46,5 | 34,9 |
| 49,5 | 40,2 |
| 52,3 | 47,0 |
| 55,9 | 49,5 |
| 59,2 | 50,1 |
| 62,6 | 52,6 |
| 65,8 | 55,9 |
| 105,4 | 56,8 |
| 108,1 | 59,4 |
| 116,9 | 64,1 |
| 117,5 | 66,8 |
| 134,7 | 105,0 |
| 136,0 | 107,1 |
| 137,3 | 116,3 |
| 140,1 | 117,4 |
| 142,6 | 135,2 |
| 150,1 | 136,1 |
| 152,6 | 137,4 |
| 165,3 | 140,8 |
| 166,0 | 143,5 |
| 175,5 | 149,3 |
| | 150,9 |
| | 168,1 |
| | 175,5 |

**Tab. 4:**

| Raman-Spektroskopie | |
|---|---|
| wasserfreie Form [cm-1] | Hydrat [cm-1] |
| 110 | 109 |
| 147 | 148 |
| 243 | 243 |
| 278 | 278 |
| 388 | 309 |
| 425 | 425 |
| 496 | 496 |
| 543 | 543 |
| 723 | 724 |
| 833 | 833 |
| 964 | 962 |
| 1031 | 1031 |
| 1191 | 1191 |
| 1267 | 1305 |
| 1305 | 1321 |
| 1320 | 1352 |
| 1354 | 1376 |
| 1376 | 1433 |
| 1433 | 1490 |
| 1491 | 1554 |
| 2891 | 1619 |
| 2922 | 1711 |
| 2957 | 2835 |
| 2991 | 2888 |
| 3020 | 2923 |
| 3054 | 2942 |
| 3069 | 2958 |
| 3082 | 2977 |
| 3088 | 2990 |
| 3110 | 3019 |
| | 3056 |
| | 3069 |
| | 3089 |
| | 3106 |

**Tab. 5:**

| FIR-Spektroskopie | |
|---|---|
| wasserfreie Form [cm-1] | Hydrat [cm-1] |
| 137 | 95 |
| 165 | 111 |
| 187 | 139 |
| 219 | 145 |
| 230 | 163 |
| 248 | 185 |
| 279 | 220 |
| 289 | 230 |
| 311 | 277 |
| 342 | 313 |
| 370 | 340 |
| 386 | 369 |
| 396 | 388 |
| 412 | 399 |
| 423 | 412 |
| 436 | 423 |
| 456 | 436 |
| 474 | 459 |
| 494 | 476 |
| | 494 |

## Patentansprüche

1. Monohydrat des CDCH der Formel 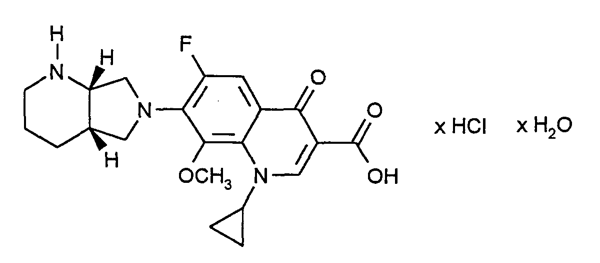 welches im ¹³C-NMR-Spektrum einen charakteristischen Peak bei 168,1 ppm und im Röntgendiffraktogramm eine Bande bei 2Θ = 26,7 besitzt.

2. Verbindung gemäß Anspruch 1 in prismatischer Kristallform.

3. Verfahren zur Herstellung des Monohydrats des CDCH gemäß Anspruch 1, **dadurch gekennzeichnet, daß** man wasserfreies CDCH mit einer zur guten Durchmischung und Hydratisierung mindestens ausreichenden Wassermenge bei Temperaturen unterhalb von 80°C bis zur Aufnahme des stöchiometrischen Kristallwassergehalts und vollständigen Kristallumwandlung behandelt, die so erhaltenen Kristalle des Monohydrats abtrennt und vorhandenes adsorbiertes Wasser entfernt.

4. Verfahren nach Anspruch 3, **dadurch gekennzeichnet, daß** man eine Suspension des wasserfreien CDCH in wäßrigen Medien bis zur vollständigen Hydratisierung und Kristallumwandlung rührt.

5. Verfahren nach Anspruch 3, **dadurch gekennzeichnet, daß** man zur Herstellung des Monohydrats in Form von Prismen wasserfreies CDCH oder CDCH-Monohydrat in Form von Nadeln in Medien mit einem stöchiometrisch ausreichendem, aber auf 10% begrenzten Wassergehalt löst und anschließend das Lösungsmittel entfernt.

6. Verfahren nach Anspruch 3, **dadurch gekennzeichnet, daß** wasserfreies CDCH einer Feuchte so lange ausgesetzt wird, bis die Kristallumwandlung quantitativ erfolgt ist.

7. Arzneimittel enthaltend Verbindungen gemäß Ansprüchen 1 und 2.

8. Antibakterielle Mittel enthaltend Verbindungen gemäß Ansprüchen 1 und 2.

9. Verwendung von Verbindungen gemäß Ansprüchen 1 und 2 bei der Herstellung von Arzneimitteln.

## Claims

1. Monohydrate of CDCH, of the formula 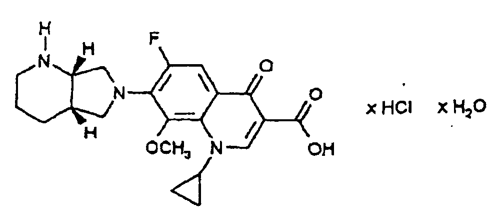 which has a characteristic peak at 168.1 ppm in the ¹³C-NMR spectrum and a band at 2Θ = 26.7 in the X-ray diffractogram.

2. Compound according to Claim 1 in the prismatic crystal form.

3. Process for the preparation of the CDCH monohydrate according to Claim 1, **characterized in that** anhydrous CDCH is treated with an amount of water which is at least sufficient for thorough mixing and hydration at temperatures below 80°C until the stoichiometric content of water of crystallization has been absorbed and conversion of the crystals is complete, the crystals of the monohydrate thus obtained are separated off and the adsorbed water present is removed.

4. Process according to Claim 3, **characterized in that** a suspension of the anhydrous CDCH in aqueous media is stirred until hydration and conversion of the crystals is complete.

5. Process according to Claim 3, **characterized in that**, to prepare the monohydrate in the form of prisms, anhydrous CDCH or CDCH monohydrate in the form of needles is dissolved in media having a water content which is stoichiometrically sufficient but limited to 10 %, and the solvent is then removed.

6. Process according to Claim 3, **characterized in that** anhydrous CDCH is exposed to humidity until the crystals have been converted quantitatively.

7. Medicaments comprising compounds according to Claims 1 and 2.

8. Antibacterial compositions comprising compounds according to Claims 1 and 2.

9. Use of compounds according to Claims 1 and 2 in the preparation of medicaments.

## Revendications

1. Monohydrate du CDCH de la formule : 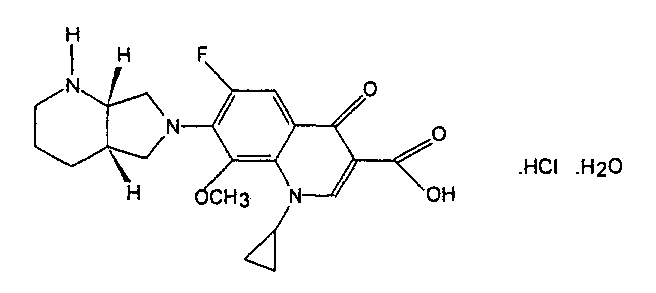 qui possède dans le spectre RMN ¹³C, un pic caractéristique à 168,1 ppm et dans le spectre de diffraction des rayons X, une bande à 2θ = 26,7.

2. Composé suivant la revendication 1, sous forme cristalline prismatique.

3. Procédé de préparation du monohydrate du CDCH suivant la revendication 1, **caractérisé en ce que** l'on traite le CDCH anhydre avec une quantité d'eau au moins suffisante pour un bon mélangeage et l'hydratation, à une température inférieure à 80°C, jusqu'à l'absorption de la teneur stoechiométrique en eau de cristallisation et transformation cristalline complète, on sépare les cristaux ainsi obtenus du monohydrate et on élimine l'eau adsorbée présente.

4. Procédé suivant la revendication 3, **caractérisé en ce que** l'on agite une suspension du CDCH anhydre dans un milieu aqueux jusqu'à hydratation complète et transformation cristalline.

5. Procédé suivant la revendication 3, **caractérisé en ce que** l'on dissout pour la préparation du monohydrate sous forme de prismes, le CDCH anhydre ou le monohydrate de CDCH sous forme d'aiguilles dans un milieu avec une teneur en eau stoechiométrique suffisante, mais limitée à 10% et ensuite, on élimine le solvant.

6. Procédé suivant la revendication 3, **caractérisé en ce que** le CDCH anhydre est exposé à de l'humidité, pendant une période telle que la transformation cristalline soit quantitativement réalisée.

7. Médicaments contenant des composés suivant les revendications 1 et 2.

8. Agents antibiotiques contenant des composés suivant les revendications 1 et 2.

9. Utilisation des composés suivant les revendications 1 et 2 pour la préparation de médicaments.
